# EUROPÄISCHE PATENTSCHRIFT

(11) **EP 0 681 849 B1**
(45) Veröffentlichungstag und Bekanntmachung des Hinweises auf die Patenterteilung: **27.08.2003**
(21) Anmeldenummer: 95250110.4
(22) Anmeldetag: 09.05.1995
(51) Int. Cl.: A61N 1/368, A61N 1/37

(54) **Vorrichtung zur Effektivitätserkennung von Herzreizimpulsen**
Device for recognizing the effectiveness of heart pacing pulses
Appareil pour la reconnaissance de l'efficacité des impulsions de stimulation cardiaque

(30) Priorität: 09.05.1994 DE 4416778
(43) Veröffentlichungstag der Anmeldung: 15.11.1995
(73) Patentinhaber: BIOTRONIK Mess- und Therapiegeräte GmbH & Co Ingenieurbüro Berlin, 12359 Berlin (DE)
(72) Erfinder: Fröhlich, Ronald, D-91056 Erlangen (DE); Bolz, Armin, D-91058 Erlangen (DE); Schaldach, Max, D-91054 Erlangen (DE)
(74) Vertreter: Christiansen, Henning, Dipl.-Ing.

(56) Entgegenhaltungen:
- EP-A- 0 313 881
- WO-A-94/12237
- US-A- 4 674 508
- US-A- 4 686 988
- US-A- 4 858 610
- US-A- 5 350 410

## Beschreibung

Die Erfindung betrifft eine Vorrichtung zur Effektivitätserkennung von Herzreizimpulsen.

Es ist bekannt, daß die Stimulationsschwelle ("threshold") eines Herzschrittmachers, d.h. die zur Erzeugung einer Erregung auf einen Reizimpuls hin nötige Reizimpulsenergie, sich im Zuge des Einwachsens der Schrittmacherelektrode(n), infolge des Einflusses von Pharmaka, durch pathologische Vorgänge im Herzen oder durch Mikrodislokationen der Elektrode(n) ändern kann. Eine einmal - etwa durch anfängliche Programmierung der Ausgangsspannung der Schrittmacherausgangsstufe - vorgenommene Einstellung der Reizimpulsenergie wird dann entweder im Hinblick auf die Sicherheit des Patienten oder auf einen batterieschonenden Betrieb des Schrittmachers und damit dessen Lebensdauer nicht mehr optimal sein.

Es ist daher ein wesentliches Leistungsmerkmal eines modernen Herzschrittmachersystems, daß bei Nachsorgeuntersuchungen die aktuelle Schwelle festgestellt und der Schrittmacher darauf neu eingestellt werden kann.

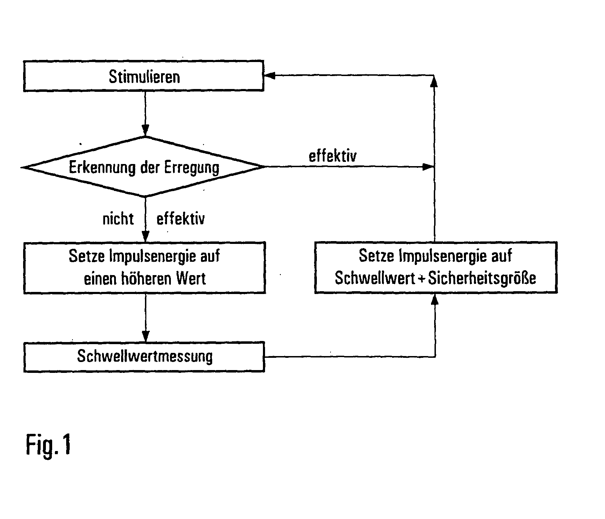

Soll eine solche Einstellung automatisch vorgenommen werden, so kann grundsätzlich nach dem in Fig. 1 gezeigten schematisierten Flußdiagramm vorgegangen werden.

Ein zur selbsttätigen Einstellung der Reizenergie fähiger Schrittmacher ist aus US 3,949,758 bekannt. Bei diesem Schrittmacher wird innerhalb eines voreingestellten Zeitfensters nach einem Stimulationsimpuls das Auftreten eines Herzaktionssignals erfaßt. Tritt ein Signal auf, wird dieses als Stimulationsantwort gewertet, tritt kein Signal auf, wird von einer Unterschreitung der Schwelle ausgegangen und die Impulsenergie erhöht. Bei dieser Vorrichtung werden zufällig in das Zeitfenster fallende spontane Herzaktionen fälschlich als Stimulationsantwort gedeutet, was zu gefährlichen Fehlsteuerungen des Schrittmachers führen kann.

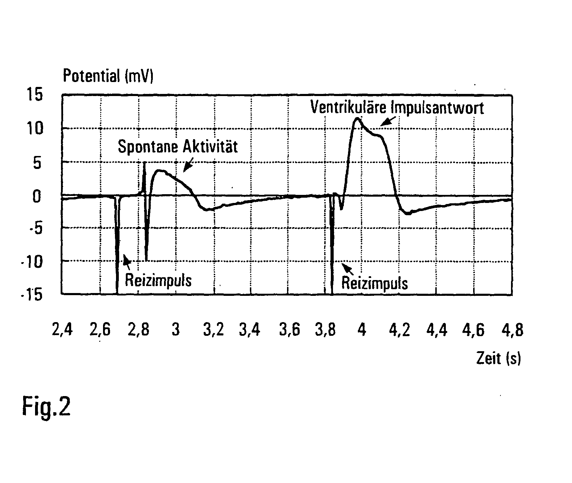

Daher ist es unbedingt erforderlich, stimulierte Herzaktionen (evozierte Potentiale) von spontanen bzw. autonomen Herzaktionen - beide sind beispielhaft in Fig. 2 dargestellt - ohne diagnostische Mitwirkung des Arztes zuverlässig zu unterscheiden.

Ein solches Schrittmachersystem, das speziell auch den Nachweis einer atrialen Erregung (P-Welle) ermöglicht, ist etwa aus US 4,686,988 bekannt. Dieses System weist eine gesonderte Elektrodenanordnung und Eingangsschaltung für atriale Elektrogramme auf, mit deren Hilfe stimulierte Herzaktionen auch kurz nach dem Reizimpuls nachgewiesen werden können und ihre Unterscheidung von spontanen Herzaktionen möglich sein soll. Zur Einstellung der Schwelle ist eine Auswertung der Signale durch den Arzt nötig, und die Einstellung erfolgt manuell durch diesen. Die zusätzlichen Elektroden bringen einen hohen Aufwand mit sich, und die Unterscheidung zwischen stimulierten und spontanen Herzaktionen allein aufgrund einer Beurteilung des Zeitabstandes zum Reizimpuls erscheint als nicht hinreichend verläßlich.

Bei einer aus US 4,674,509 bekannten Anordnung wird versucht, durch Aussendung von Impulspaaren, von denen ein Impuls mit Sicherheit eine Erregung bewirkt und der andere nicht, und eine eine Subtraktion der Impulsantwort-Signale umfassende Auswertung das auf einen wirksamen Impuls zurückzuführende Herzaktionspotential von Artefakt-Anteilen zu isolieren. Eine Unterscheidung von konkurrierend auftretenden spontanen Herzaktionen ist damit nicht möglich, und die Anordnung ist als Hilfsmittel für die Diagnose und manuelle Einstellung der Impulsenergie durch den Arzt gedacht.

In US 4,858,610 ist eine Vorrichtung zum automatischen Nachweis evozierter Potentiale beschrieben, bei der zu deren Erfassung eine gesonderte Ringelektrode und Eingangsstufe vorgesehen ist. Die Erfassung der natürlichen bzw. spontanen Herzaktivität erfolgt in einem bipolaren Modus mittels dieser Ringelektrode und der Stimulationselektrode, allerdings lediglich, um ggf. eine Inhibition des Reizimpulses zu bewirken und nicht zum Zwecke der Unterscheidung zwischen spontanen und stimulierten Herzaktionen. Bei dieser Vorrichtung wird über die Wahl eines Messzeitfensters ein Ausblenden natürlicher Herzaktionen versucht. Fallen jedoch spontane Herzaktionen in das Zeitfenster - was keineswegs auszuschließen ist - muss es zu Fehlinterpretationen kommen. Das System ist daher für eine automatische Schwellwertbestimmung und Impulsenergieeinstellung ungeeignet.

Die WO 94/12237, die eine Priorität einer US-Anmeldung vom 23. November 1992 beansprucht, betrifft ein autocapture-System für implantierbare Schrittmacher. Innerhalb eines vorbestimmten Zeitraumes wird ein Messpunkt erfasst und durch Amplitudendiskriminierung mit vorbestimmten Referenzspannungen verglichen. Der Vergleich liefert ein sogenannten Differenzsignal, das in der weiteren Schaltungslogik zur Beeinflussung der Steuerungsparameter der Schrittmacheranordnung genutzt wird.

Die US 4,674,508, gemäß Oberbegriff von Anspruch 1, betrifft einen Herzschrittmacher, dessen Energieverbrauch durch automatische Verifikation evozierter Kontraktionen gesenkt werden soll. Dabei werden empfangene Polarisationssignale mit Referenzsignalen verglichen und liefern ein Differenzsignal. Das Differenzsignal wird durch Zeitintegration weiterverarbeitet, und anhand vorgegebener Werte klassifiziert, wobei eine Aussage über die Effektivität des Herzreizimpulses erfolgt.

Die EP 0 313 881 betrifft einen ratenadaptiven Herzschrittmacher, bei dem mittels einer geeigneten Steuerung ein Betriebsmodus und/oder eine Stimulationsrate vorgegeben wird. Zur Steuerung der Stimulationsrate wird vorgeschlagen, zunächst wie allgemein üblich einem Sensorsignal eine Stimulationsrate zuzuordnen. Die Zuordnung erfolgt aber erst dann, wenn das Signal eine bestimmte höchste Leserate unterschreitet. Dieser Herzschrittmacher weist eine Hystereseschaltung auf, um Fusion - Beats zu vermeiden.

Der Erfindung liegt die Aufgabe zugrunde, ein Gerät der eingangs genannten Gattung anzugeben, mit dem eine sichere Erkennung stimulierter Herzaktionen bzw. evozierter Potentiale und darauf beruhend eine zuverlässige, automatisierbare Effektivitätserkennung möglich ist. In einer bevorzugten Ausführung soll an Schrittmacher mit automatischer Einstellung der Impulsenergie angegeben werden.

Die Erfindung schließt den Gedanken ein, eine Unterscheidung zwischen stimulierten und spontanen Herzaktionen primär aufgrund der sich unterscheidenden Signalform vorzunehmen. Unter Beachtung der typischen Signalformen (siehe Fig. 2), die sich in der Frequenzdomäne dadurch auszeichnen, daß das evozierte Potential (speziell die ventrikuläre Impulsantwort - VER) bei niedrigen Frequenzen Anteile mit hoher Amplitude hat, während die autonome Aktivität bei höheren Frequenzen Anteile mit hoher Amplitude hat, werden dazu vor allem folgende Vorgehensweisen vorgeschlagen:
a) eine zur Aufteilung des Gesamt-Frequenzspektrums in charakteristische Bereiche geeignete Filterung mit anschließender Amplitudendiskriminierung,
b) eine Frequenzanalyse (etwa durch schnelle Fourier-Transformation - FFT), speziell unter Einschluß eines Vergleiches mit vorgegebenen Mustern, oder
c) ein Sampling mit genügend vielen Meßpunkten in einem hinreichend breiten Meßzeitfenster mit anschließendem Vergleich mit einer vorgegebenen Referenzspannung.

Die Frequenzanalyse nach b) mit anschließender Bewertung kann einen Amplitudenvergleich der Signalanteile bei mindestens zwei vorbestimmten Frequenzen oder in mindestens zwei Frequenzbereichen einschließen.

Die Amplitudendiskriminierung nach c) kann eine Unterscheidung zwischen spontaner Herzaktion und evoziertem Potential anhand der absoluten oder relativen Häufigkeit des Auftretens von Amplitudenwerten unterhalb der Referenzspannung einschließen; es kann etwa schon das Auftreten eines unterhalb der Referenzspannung liegenden Signalwertes als Kennzeichen für das Vorliegen einer spontanen Herzaktion gelten.

Die Messungen erfolgen in einem Meßzeitfenster mit vorbestimmter Breite - nach Untersuchungen der Erfinder vorzugsweise etwa 60 ms - , dessen Beginn - vorzugsweise innerhalb des Bereiches von 150 bis 250 ms nach dem Stimulationsimpuls - programmierbar ist.

Die Abtastrate für die einzelnen Messungen innerhalb des Meßzeitfensters ist vorzugsweise größer als 100 Hz, und die Referenzspannung ist etwa zwischen 1 und 15 mV in Schritten von 1 mV einstellbar.

Zur Ausblendung von Signalanteilen des Reizimpulses wird eine Signalaustastung - vorzugsweise für 50 ms - und zur Ausfilterung von Störfrequenzanteilen vor der Auswertung eine Bandfilterung mit einem Durchlaßbereich von etwa 0,5 bis 10 Hz vorgenommen.

Das Auftreten von hinsichtlich der Auswertung besonders problematischen kombinierten Herzaktionspotentialen ("fusion beats") wird in zweckmäßiger Weise von vornherein durch einen geeigneten Stimulationsmodus verhindert:

In einer atrium-synchronen Betriebsweise des Schrittmachers (dem DDD- oder VDD-Modus) wird dazu eine unterhalb der natürlichen AV-Überleitungszeit liegende AV-Verzögerung (beispielsweise 100 ms) für den Reizimpuls eingestellt und das Meßzeitfenster vor Ablauf der natürlichen AV-Überleitungszeit geschlossen, so daß noch keine spontane Erregungskomponente übergeleitet worden und somit im Meßsignal enthalten sein kann.

Im VVI-Modus wird bei Nichterfassung eines evozierten Potentials (Aussage: keine Erregung - "no capture") eine erste Folge von Reizimpulsen mit vergrößertem Impulsabstand (verringerter Rate) ausgesandt und anschließend der Impulsabstand wieder sprunghaft auf den ursprünglichen Wert verringert (die Rate erhöht) und eine zweite Folge von Reizimpulsen ausgesandt, wobei der vorbestimmte Zeitraum der Aufnahme und Auswertung innerhalb der zweiten Folge - vorzugsweise zu deren Beginn - festgelegt wird.

Beim Verfahrensablauf zur automatischen Einstellung der Impulsenergie - speziell der Impulsamplitude bzw. Ausgangsspannung des Schrittmachers - werden in Abhängigkeit vom Betriebsmodus jeweils entsprechend dem Verfahren zur Effektivitätserkennung spezifische Vorkehrungen zur Vermeidung der Entstehens von "fusion beats" getroffen.

In zweckmäßiger Ausbildung des Ablaufes zur Gewährleistung einer hohen Patientensicherheit wird bei Feststellung fehlender Effektivität eines ausgesandten Stimulationsimpulses ein folgender Impuls mit auf einen ersten Sicherheits-Energiewert (beispielsweise einen Ausgangsspannungswert von 4,8 V) erhöhter Impulsenergie ausgesandt und wiederum die Effektivität festgestellt und bei erneuter Feststellung fehlender Effektivität ein nächster Herzreizimpuls mit auf einen zweiten Sicherheits-Energiewert (beispielsweise die maximale Ausgangsspannung des Schrittmachers von 9,6 V) erhöhter Impulsenergie ausgesandt.

Um Verfälschungen der Messungen durch "fusion beats" sowie die im Ausgangskondensator des Schrittmachers noch gespeicherte Ladung bei atrium-synchronem Betrieb zu vermeiden, wird bei Feststellung fehlender Effektivität des anfänglich ausgesandten Impulses eine unterhalb der natürlichen AV-Überleitungszeit liegende AV-Verzögerung (etwa 100 ms) eingestellt und vor dem folgenden Impuls mit erhöhter Impulsenergie zunächst mindestens ein weiterer Impuls mit der anfänglich eingestellten Energie ausgesandt und für diesen die Effektivität festgestellt.

Um einen korrekten Verfahrensablauf speziell auch bei Vorliegen eines 2:1-AV-Blockes zu sichern, wobei jede zweite ventrikuläre Impulsantwort inhibiert ist, wird bei Ausfall des ersten Impulses nach der Neueinstellung der AV-Verzögerung der nächstfolgende nicht-inhibierte Impuls abgewartet und dessen Effektivität festgestellt.

In zweckmäßiger Wahl von Verfahrensparametern erfolgt bei jedem Schritt der Verminderung der Impulsenergie jeweils - ausgehend vom anfänglich eingestellten Wert oder (falls dieser keine Erregung bewirkt hatte) einem der Sicherheitswerte - eine Verringerung der Ausgangsspannung um 200 mV, und die nach der Bestimmung der Reizschwelle gewählte Impulsenergie wird mit einem Sicherheits-Summanden von 1 V eingestellt.

Eine Vorrichtung zur Durchführung des Verfahrens weist eine von einer Schrittmachereingangsschaltung zur Erfassung eines intrakardialen EKG getrennte, vor der Anti-aliasing-Schaltung des Schrittmachereinganges mit der Aufnahmeelektrode verbundene Aufnahme- und Auswertungsschaltung und eine Steuereinheit zur Steuerung des Verfahrensablaufes auf und ist vorzugsweise als eigenständige integrierte Schaltung ausgeführt und implantierbar, wobei sie von außerhalb des Körpers, insbesondere über einen Reed-Schalter, aktivierbar ist.

Die Aufnahme- und Auswertungsschaltung umfaßt insbesondere
- eine Austastschaltung zum Ausblenden von Signalen unmittelbar nach dem Herzreizimpuls,
- einen Vorverstärker
- einen programmierbaren Meßverstärker,
- ein Bandfilter mit einem Durchlaßbereich von 0,5 bis 10 Hz,
- eine Vergleichereinheit zur Amplitudendiskriminierung des Meßsignals,
- einen A/D-Wandler und
- eine logische Verarbeitungseinheit zur Analyse der Vergleichsergebnisse entsprechend dem Verfahrensablauf.

Die Steuerung der Vorrichtung kann mindestens teilweise über den Mikroprozessor des Schrittmachers erfolgen, wobei auch dessen Telemetriesystem zur Durchführung des Verfahrens sowie zur Sicherstellung der Informationsübermittlung an einen Arzt und von Eingriffsmöglichkeiten für diesen genutzt werden kann. Der separate Test-Schaltkreis ist dann über einen Testkanal mit dem Schrittmacher verbunden.

Um den Zeitabstand zum Stimulationsimpuls verfügbar zu haben, ist eine. Signalverbindung zum Schrittmacher zur Übermittlung eines die Stimulationsimpulse repräsentierenden Signals oder ein eigenständiger Meßkanal zur Erfassung der Stimulationsimpulse vorgesehen. Weiterhin ist eine Einrichtung zur Triggerung des Verfahrensablaufes in Reaktion auf die erfaßten Stimulationsimpulse vorgesehen.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
Figur 1 ein schematisches Flußdiagramm eines Verfahrensablaufes zur automatischen Einstellung der Impulsenergie bei einem Schrittmacher,
Figur 2 eine grafische Darstellung eines ventrikulären Elektrogramms, das eine spontane Herzaktion und ein evoziertes Potential in Abhängigkeit von der zeit zeigt,
Figur 3 eine grafische Darstellung eines evozierten Potentials in Abhängigkeit von der Zeit mit einem Meßzeitfenster für die Effektivitätserkennung,
Figuren 4a bis 4c ein Flußdiagramm eines Verfahrens zur automatischen Einstellung einer Schrittmacherimpulsamplitude im DDD-Modus eines Herzschrittmachers gemäß einer Ausführungsform der Erfindung,
Figuren 5a bis 5d ein Flußdiagramm eines Verfahrens zur automatischen Einstellung einer Schrittmacherimpulsamplitude im VVI-Modus eines Herzschrittmachers gemäß einer weiteren Ausführungsform der Erfindung,
Figur 6 eine schematische Darstellung der Rate bei einem Stimulations- und einem spontanen Herzrhythmus in Abhängigkeit von der Zeit bei einer Ausgestaltung des Verfahrens nach Fig. 5a bis 5c,
Figur 7 ein vereinfachtes Blockschaltbild einer Vorrichtung zur Durchführung des Verfahrens in einer Ausführungsform sowie einer Schrittmachereingangsschaltung und
Figur 8 eine schematische Darstellung des Zusammenwirkens einer Vorrichtung zur Durchführung des Verfahrens mit einem programmierbaren Herzschrittmacher.

In Fig. 1 ist schematisch ein Verfahrensablauf für die automatische Einstellung der Impulsenergie eines Stimulators für reizbares Körpergewebe - beispielsweise eines Herzschrittmachers - dargestellt.

Das Verfahren beginnt, indem mit einer voreingestellten Energie stimuliert und anschließend festgestellt wird, ob die Stimulation zu einer Erregung geführt hat oder nicht. War sie effektiv, wird weiterhin mit der eingestellten Energie stimuliert. War sie nicht effektiv, wird die Impulsenergie auf einen (wesentlich) höheren Wert gesetzt und anschließend eine (weiter unten genauer erläuterte) Prozedur zur Bestimmung der Reizschwelle ausgeführt. Der im Ergebnis dieser Prozedur erhaltene Schwellwert wird mit einer Sicherheitsgröße (in der Figur einem Summanden) verarbeitet und so der Wert erhalten, auf den die Impulsenergie für die weitere Stimulierung eingestellt wird.

Der beispielhaft dargestellte Ablauf ist im Ansatz sicherheitsorientiert, d.h. solange eine Stimulation mit dem voreingestellten Energiewert zu einer Erregung führt, wird mit diesem Energiewert weitergearbeitet, ohne eine mögliche Senkung der Impulsenergie zu prüfen. Steht bei der Einstellung die Minimierung des Batterieverbrauchs und damit die Maximierung der Lebensdauer im Mittelpunkt, ist der Ablauf entsprechend zu modifizieren.

Fig. 2 zeigt beispielartig ein intraventrikulär aufgenommenes Elektrogramm, in dessen linkem Bereich ein Stimulationsimpuls mit sich anschließender autonomer bzw. spontaner Herzaktion und in dessen rechtem Bereich ein Stimulationsimpuls mit auf diesen folgender Impulsantwort (evoziertem Potential)in Abhängigkeit von der Zeit gezeigt ist.

Es ist zu erkennen, daß die autonome Herzaktion ein Signal mit sich deutlich vom evoziertem Potantial unterscheidender Signalform liefert, wobei insbesondere die starke Schwankung der Amplitude im Einsetzbereich der spontanen Herzaktion ins Auge fällt.

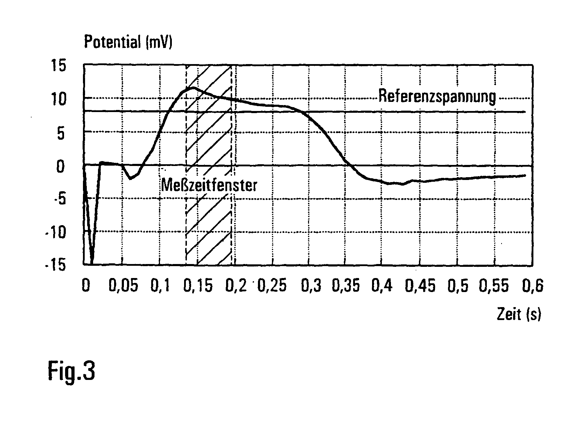

Fig. 3 bezeichnet mit der Angabe eines (schraffierten) Zeitfensters einen Abschnitt auf der - mit anderem Maßstab als in Fig. 2 dargestellten - Zeitachse, der zur Feststellung von Unterschieden in der Signalform der beiden in Fig. 2 gezeigten Signale besonders geeignet ist.

Bei der nachfolgenden Beschreibung von Ausführungsbeispielen wird davon ausgegangen, daß in diesem Zeitfenster mit einer Breite von 60 ms und im Bereich von knapp unter 150 ms bis 250 ms (in der Figur auf ca. 130 ms eingestellt) nach einem Stimulationsimpuls ein Sampling der an der Spitze einer Schrittmacherleitung aufgenommenen, einer Bandfilterung mit einem Durchlaßbereich von 0,5 bis 10 Hz unterworfenen, Signale mit einer Abtastfrequenz von minimal 100 Hz erfolgt. Die erhaltenen Spannungswerte werden einer Amplitudendiskriminierung mit einer im Bereich von 1 bis 15 mV einstellbaren Referenzspannung unterzogen. Eine Stimulation wird als erfolgreich (als Erregung - "capture") angesehen, wenn alle im Zeitfenster aufgenommenen Spannungswerte über der programmierten Referenzspannung liegen, hingegen als nicht erfolgreich ("no capture"), wenn mindestens einer der Werte unterhalb der Referenzspannung liegt.

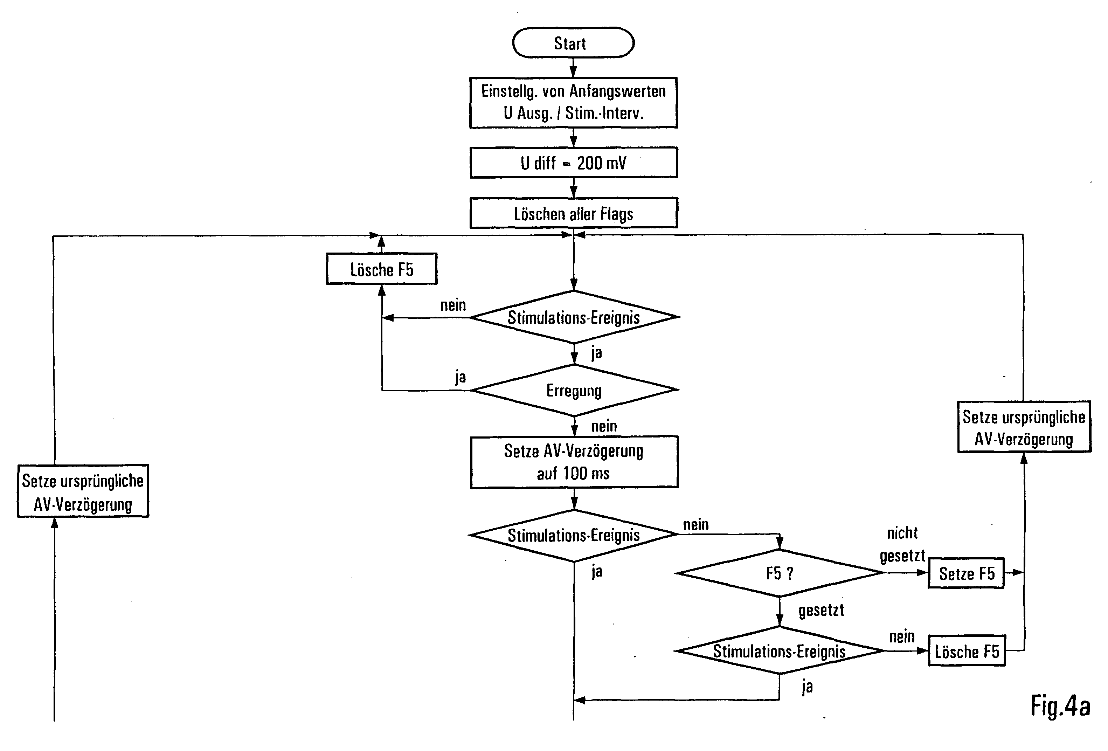

In den Figuren 4a bis 4c ist das Flußdiagramm eines Verfahrens zur automatischen Einstellung der Schrittmacherimpulsamplitude im DDD-Modus eines Herzschrittmachers gemäß einer Ausführungsform der Erfindung gezeigt. Das Verfahren wird nachfolgend kurz beschrieben, ohne daß alle aus dem Diagramm ohne weiteres ersichtlichen Schritte nochmals im einzelnen wiederholt werden. Im Programmablauf werden vier Kennzeichen (Flags) verwendet.

Zu Beginn werden vom Arzt Anfangsparameter (insbesondere eine Ausgangsspannung und eine Impulsrate und ein Spannungsdifferenzbetrag, um den die Ausgangsspannung in den anchfolgenden Schritten jeweils zu verringern ist - in Fig. 4a mit U diff = 200 ms angegeben) programmiert, der Programmiermagnet wird entfernt, und es werden alle Flags (die nachfolgend bzw. im Diagramm erläutert sind) gelöscht.

Wird auf ein Stimulations-Ereignis hin eine Erregung nachgewiesen, wird die Schleife geschlossen, und der Schrittmacher arbeitet mit den programmierten Parametern.

Wird keine Erregung nachgewiesen, wird die AV-Verzögerung auf 100 ms eingestellt, um - wie weiter oben erläutert - "fusion-beats" auszuschließen, und unter Zuhilfenahme des Flag F5 wird ein Teilablauf abgearbeitet, der zur Vermeidung von Fehlinterpretationen bei möglichem Vorliegen eines 2:1-AV-Blocks dient. (Andere Arten von AV-Blöcken, etwa der 4:1-Block, sind nicht lebensbedrohlich, so daß daraus resultierende Fehlaussagen unkritisch sind.) Dazu wird das Flag F5 gesetzt, wenn auf einen ersten Impuls keine Erregung festgestellt wurde und der nächste Impuls inhibiert ist. Daraufhin wird im Algorithmus der Impuls unmittelbar nach dem inhibierten daraufhin untersucht, ob er von einer Erregung gefolgt ist oder nicht. Wenn ja, wird - nach Löschen von F5 - wieder die ursprüngliche AV-Verzögerung eingestellt und der vorhergehende Ablauf wiederholt.

Wenn nein, wird die Ausgangsspannung auf einen ersten Sicherheitswert U Ausg von 4,8 V und danach, falls noch immer keine Erregung erreicht wurde, auf 9,6 V heraufgesetzt.

Für eine vom Arzt vorgenommene Messung der Reizschwelle ist hier ein gesonderter Zugang vorgesehen, über den - unter Setzung eines Flag F4 - die Ausgangsspannung getrennt vom übrigen Ablauf programmiert und in 100-mV-Schritten variiert werden kann.

Im Programmablauf wird dann wieder die ursprüngliche AV-Verzögerung eingestellt und es werden zehn Ereignisse beim (ersten oder zweiten) Sicherheits-Amplitudenwert abgewartet. Dann wird mit jeweils um die vorgegebene Differenzspannung U diff abgesenkter Amplitude U test in der entsprechend der Lage der Reizschwelle erforderlichen Anzahl von Durchläufen der eigentliche Reizschwellentest ausgeführt. Dabei werden die weiteren Flags F2 und F3 eingesetzt, wobei mit F2 das Abwarten jeweils zweier erfolgreicher Nachweiszyklen bei jedem Wert der Testamplitude vor einer weiteren Reduzierung und mit F3 das Abwarten zweier nicht mehr erfolgreicher Nachweiszyklen vor der Fixierung der aktuellen Amplitude als Reizschwelle realisiert wird.

Nach Abschluß des Reizschwellentests wird mit der um 1V erhöhten letzten Testspannung als aktueller Ausgangsspannung weiterstimuliert. Falls der Ablauf einer Untersuchung durch den Arzt folgte (Flag F4 gesetzt), wird der Ablauf über einen separaten Ausgang unter Einstellung von U Ausg auf 4,8 V beendet.

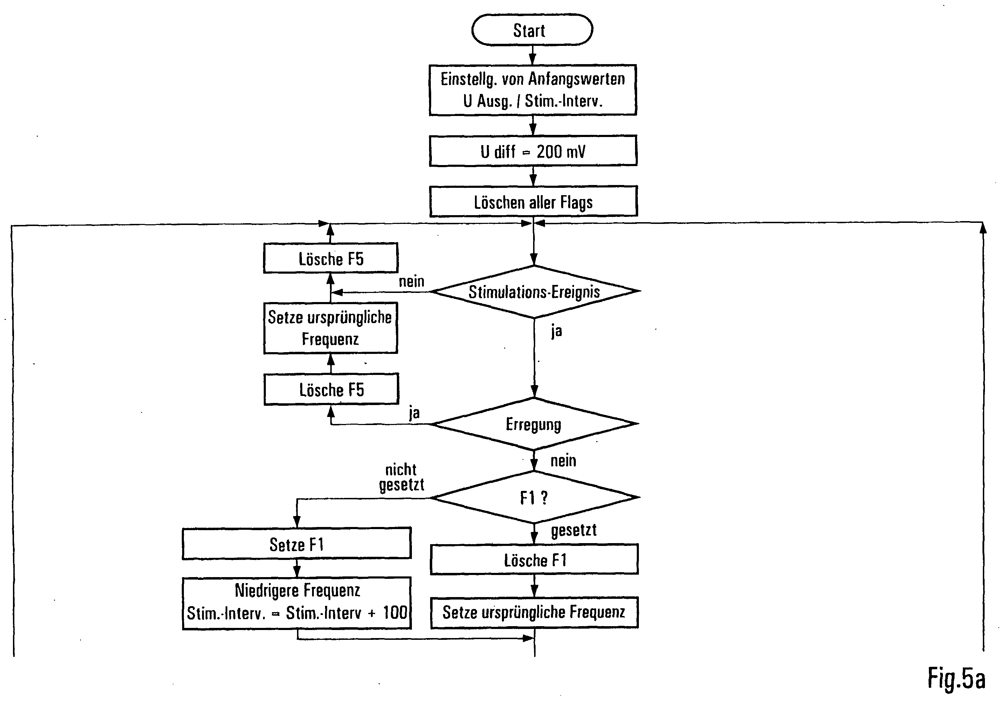

In den Figuren 5a bis 5c ist das Flußdiagramm eines zu dem in Fig. 4a bis 4c gezeigten Verfahren ähnlichen Verfahrens im VVI-Modus eines Herzschrittmachers gezeigt. Nachfolgend wird nur auf die Unterschiede dieses Verfahren gegenüber dem vorbeschriebenen hingewiesen, wobei auch hier die aus dem Diagramm ersichtlichen Schritte nicht im einzelnen wiederholt werden. Im Programmablauf werden fünf Kennzeichen (Flags) verwendet.

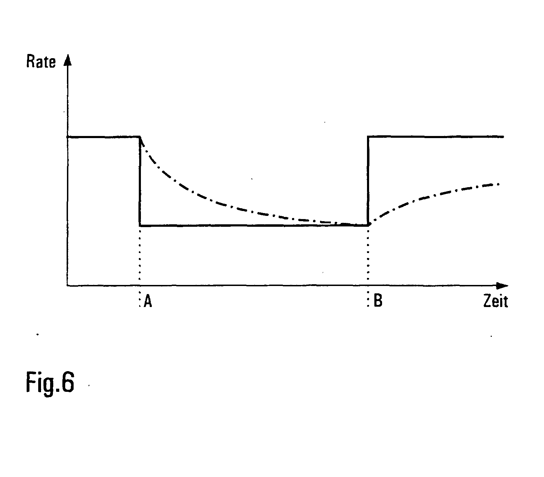

Wird auf ein Stimulationsereignis hin keine Erregung nachgewiesen, wird zur Realisierung eines weiter unten unter Bezugnahme auf Fig. 6 genauer erläuterten Vorgehens unter Zuhilfenahme des Flag F1 die Stimulationsfrequenz zunächst verringert und dann wieder auf den ursprünglichen Wert erhöht, um - wie weiter oben für diesen Betriebsmodus bereits erwähnt - "fusion-beats" auszuschließen, und unter Zuhilfenahme der Flag F5 werden wieder Fehlinterpretationen bei möglichem Vorliegen eines 2:1-AV-Blocks vermieden. (Dies wurde oben mit Variation der AV-Verzögerung beschrieben und wird hier analog unter Variation der Stimulationsfrequenz bzw. -rate ausgeführt.)

Der weitere Ablauf entspricht dem Verfahren im DDD-Modus, wobei an die Stelle einer Variation der AV-Verzögerung jeweils eine solche der Stimulationsfrequenz tritt und zwischen die Schritte "Lösche F2" und "F3" im Hauptstrang die Wiedereinstellung der gewählten Spannung U Ausg und der ursprünglichen Frequenz sowie das Abwarten eines Stimulationsereignisses eingeschoben sind.

Fig. 6 zeigt in einer schematischen Darstellung, wie im VVI-Betriebsmodus - in dem bekanntlich nicht atriumsynchron stimuliert wird und es daher keine Möglichkeit gibt, das Zusammenfallen von stimulierten und spontanen Herzaktionen durch Verkürzung der AV-Verzögerung zu verhindern - ein solches Zusammenfallen ("fusion beats") verhindert werden kann:

Wenn bei einer bestimmten voreingestellten Stimulationsenergie und -rate keine Impulsantwort beobachtet wird, wird zu einem Zeitpunkt A die Stimulationsrate (in der Figur mit der durchgezogenen Linie dargestellt) um einen vorbestimmten Betrag verringert und mit der neuen Rate für einen bestimmten Zeitraum weiter stimuliert. Der Zeitraum wird so bemessen, daß die natürliche Sinusfrequenz (in der Figur mit einer strichpunktierten Linie bezeichnet) sich dem neuen Wert der Stimulationsrate anpassen kann. Danach wird zum Zeitpunkt B die Stimulationsrate wieder auf den ursprünglichen Wert heraufgesetzt. Da die natürliche Herzrate wiederum - wie in der Figur am langsamen Ansteigen der strichpunktierten Linie zu erkennen - vezögert nachfolgt, ist nach dem Heraufsetzen ein Zeitbereich von mehreren Impulsperioden verfügbar, in dem spontane Herzaktionen mit geringerer Frequenz als evozierte Potentiale auftreten, womit ein ständiges Zusammenfallen beider innerhalb des Meßzeitfensters sicher verhindert wird.

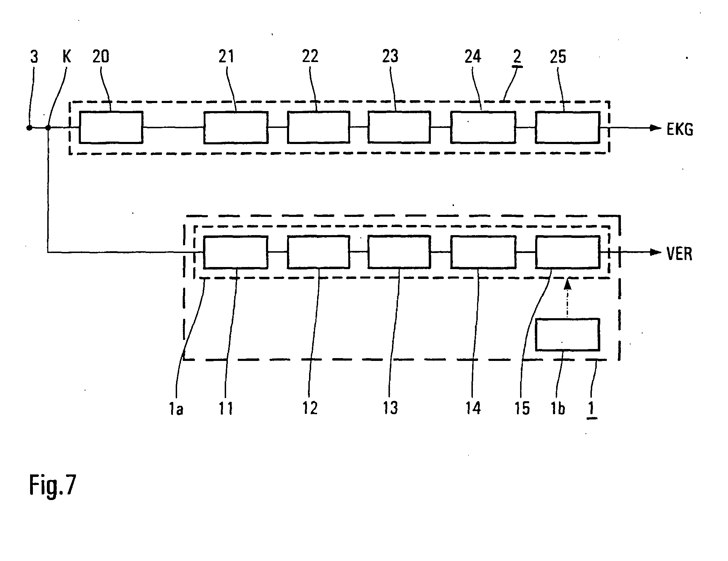

Fig. 7 ist ein vereinfachtes Blockschaltbild der wesentlichsten Elemente einer Vorrichtung 1 zur Durchführung des erfindungsgemäßen Verfahrens in einer Ausführungsform, gezeigt zusammen mit einer Schrittmachereingangsschaltung 2.

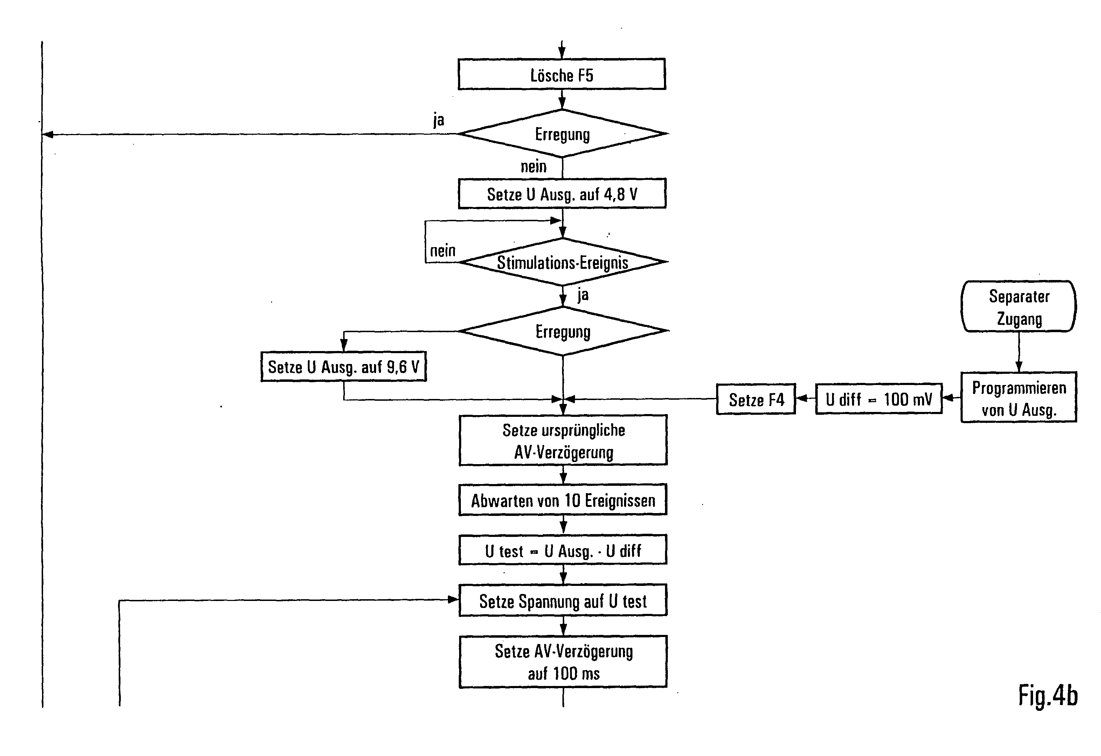

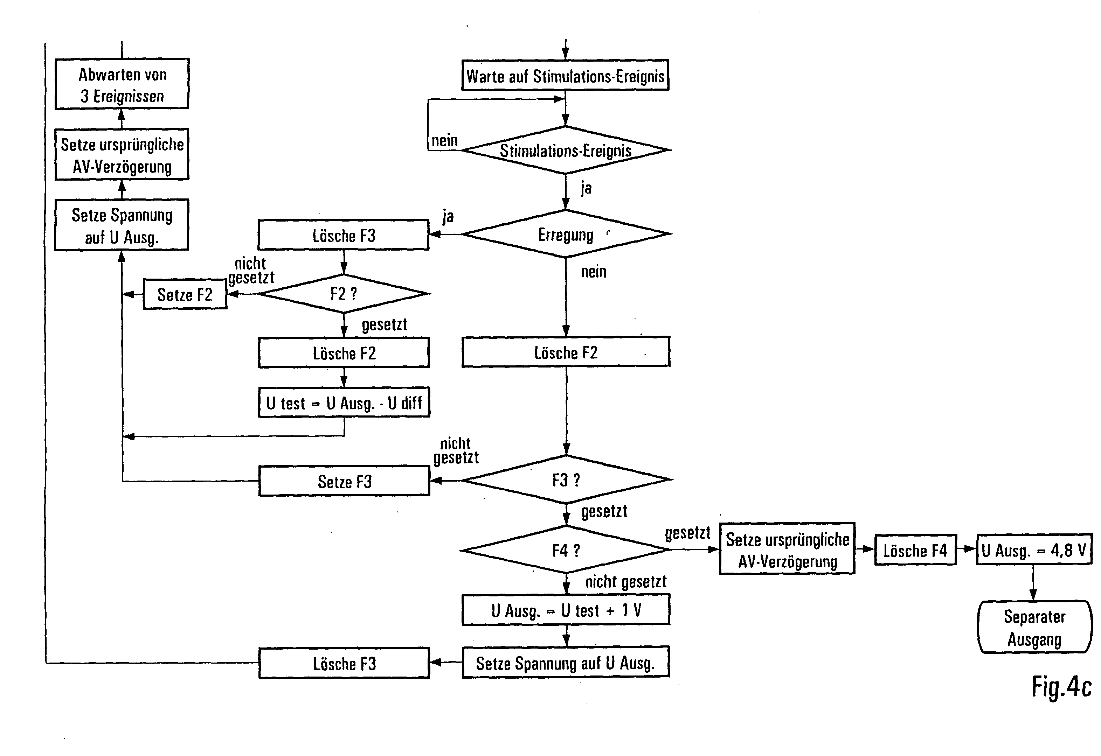

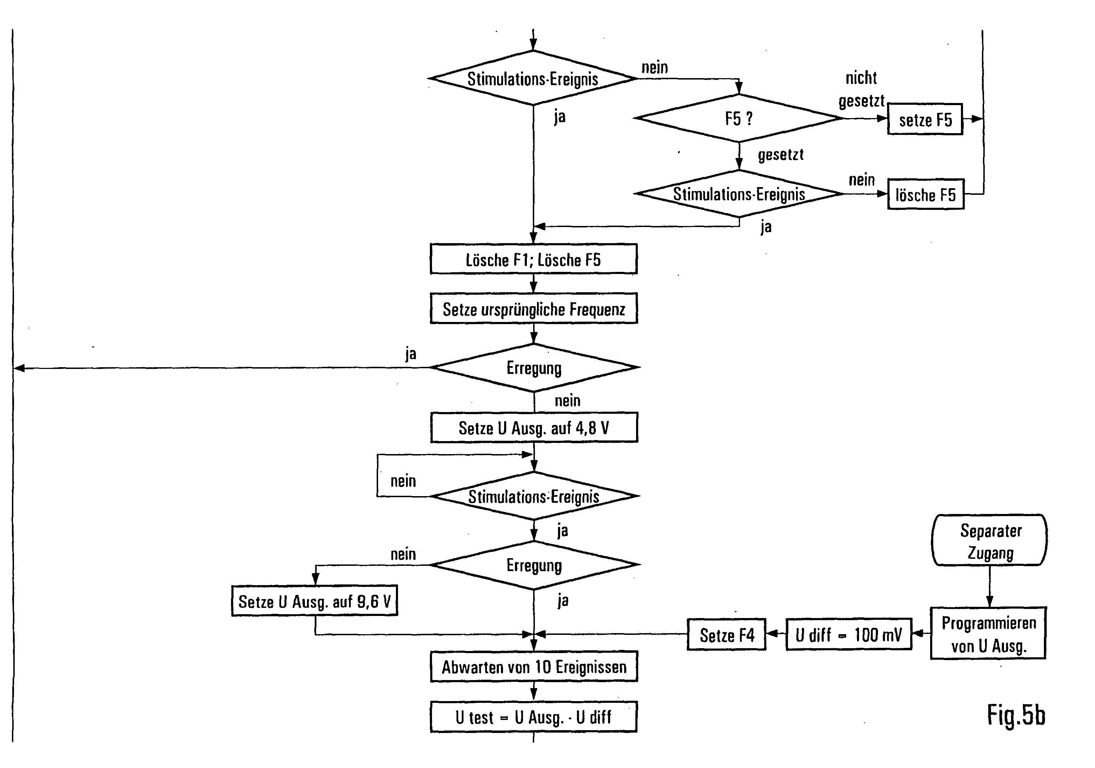

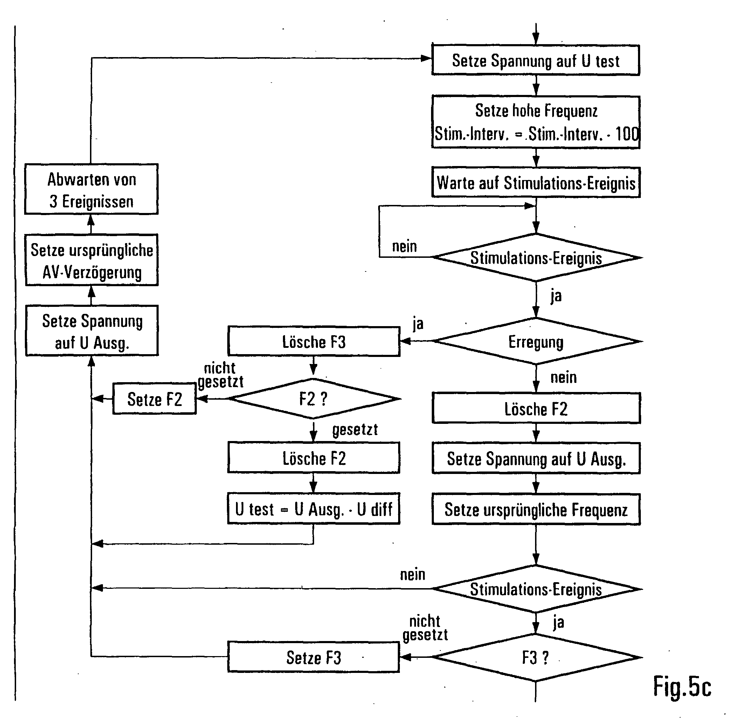

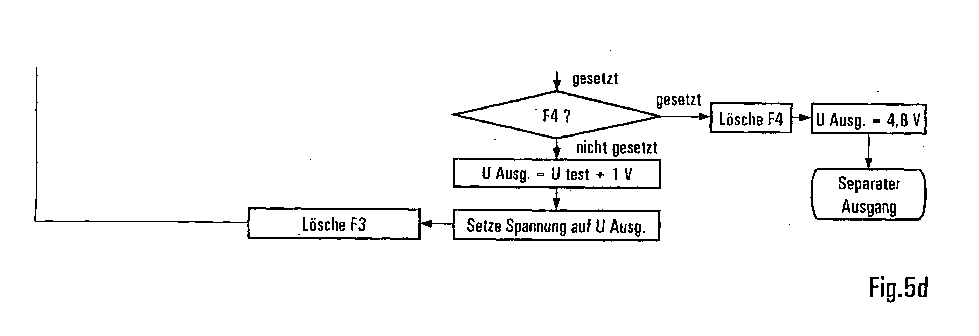

Die Vorrichtung 1 zur Effektivitätserkennung weist eine an einem Knoten K mit einer Schrittmacherleitung 3 verbundene Aufnahme- und Auswertungsschaltung 1a und eine Steuereinheit 1b auf. Die Aufnahme- und Auswertungsschaltung 1a weist einen Austast- bzw. Blankingschalter 11, dessen Austastperiode auf mindestens 50 ms eingestellt ist, einen mit dessen Ausgang verbundenen Vorverstärker 12, einen mit dessen Ausgang verbundenen Verstärker mit programmierbarer Verstärkung 13, ein mit dessen Ausgang verbundenes Bandfilter 14 mit einem Durchlaßbereich von etwa 0,5 bis 10 Hz und eine mit dessen Ausgang verbundene Diskriminator- bzw. Vergleichereinheit 15 auf, an deren Ausgang ein das vorliegen oder Nichtvorliegen einer ventrikulären Impulsantwort kennzeichnendes Signal VER bereitsteht. Dieses ist entsprechend dem gewählten Verfahrensablauf - vgl. beispielsweise Fig. 4 oder Fig. 5 - (vorzugsweise in digitaler Form) weiterzuverarbeiten. Die Weiterverarbeitung wird ebenso wie der Betrieb der Eingangsstufe - darunter insbesondere die Einstellung des Meßzeitfensters und der Referenzspannung - von der Steuereinheit 1b gesteuert

Die Schrittmachereingangsstufe 2, deren Eingang ebenfalls über den Knoten K mit der Schrittmacherleitung verbunden ist, weist eine Anti-Aliasing-Schaltung 20, einen Austast- bzw. Blankingschalter 21, einen mit dessen Ausgang verbundenen Vorverstärker 22, einen mit dessen Ausgang verbundenen programmierbaren Verstärker 23, eine mit dessen Ausgang verbundene Filterstufe 24 mit den für eine EKG-Eingangssschaltung üblichen Filtergrößen und eine mit deren Ausgang verbundene Vergleichereinheit 25 auf, an deren Ausgang ein herkömmliches EKG-Signal ECG bereitsteht. Ihr Aufbau entspricht also äußerlich weitgehend demjenigen der separaten Eingangsstufe 1, durch andere Einstellung der Filter- und Verstärkungsparameter ergibt sich jedoch eine abweichende Funktion.

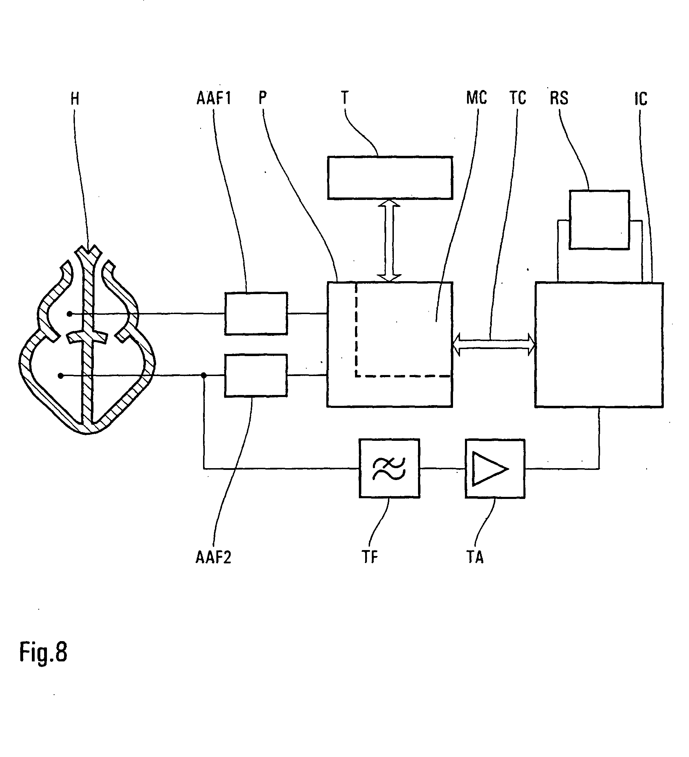

Figur 8 ist eine schematische Darstellung des Zusammenwirkens einer mit einer integrierten Schaltung IC realisierten Vorrichtung zur automatischen Einstellung der Impulsenergie ähnlich der Vorrichtung nach Fig. 7 mit einem programmierbaren Zweikammer-Herzschrittmacher P.

Der Schrittmacher P steht über je eine im Atrium und im Ventrikel eines Herzens H angeordnete Elektrode mit dem reizbaren Herzgewebe der jeweiligen Kammer in Verbindung und kann dort Reizimpulse anlegen sowie - jeweils über eine Anti-Aliasing-Einheit AAF1 bzw. AAF2- intrakardiale Elektrogramme aufnehmen. Er weist eine Mikroprozessorsteuerung MC auf und steht weiterhin wahlweise über eine Telemetrieeinheit T mit einem behandelnden Arzt in Verbindung, der über diese Einheit eine Programmierung des Schrittmachers vornehmen und von diesem Daten erhalten kann.

Die Vorrichtung zur Effektivitätserkennung und automatischen Impulsenergieeinstellung ist in diesem Beispiel verteilt realisiert und umfaßt eine Filterbaugruppe TF, eine Verstärkerbaugruppe TA, die Auswertungs-Baugruppe IC und Funktionen der Schrittmacher-Steuereinheit MC, mit der die Auswertungs-Baugruppe IC über einen Testkanal TC verbunden ist. Über diesen werden Programmstrings vom Mikroprozessor MC und auch die Abgabe von Stimulationsimpulsen kennzeichnende Signale vom Schrittmacher P an den IC übertragen, wenn ein Reedschalter RS geöffnet und ein die Durchführung der automatischen Impulsenergieeinstellung kennzeichnendes Bit gesetzt ist.

## Patentansprüche

1. Schrittmacheranordnung mit Mitteln (P) zur Aussendung eines Herzreizimpulses mit vorbestimmter Impulsenergie an ein Herz, Mitteln (1a; TF; TA; IC) zur Aufnahme und Auswertung eines Herzaktionspotentials in Abhängigkeit von der Zeit im Herzen innerhalb eines vorbestimmten Zeitraumes nach der Aussendung des Herzreizimpulses, wobei der Nachweis einer stimulierten Herzaktion die Effektivität des ausgesandten Herzreizimpulses und deren Ausbleiben fehlende Effektivität des Herzreizimpulses anzeigt, und einer Steuereinheit (1b; IC; MC), wobei die Mittel zur Aufnahme und Auswertung des Herzaktionspotentials die intraventrikuläre Aufnahme mehrerer Spannungswerte in Zeitabständen innerhalb eines im vorbestimmten Zeitraum festgelegten Messzeitfensters und zu einem Vergleich mit einem vorbestimmten Signalverlauf unter Einschluss einer Amplitudendiskriminierung des Signals in der Zeit- oder Frequenzdomäne bezüglich einer vorbestimmten Referenzspannung mit anschließender Bewertung, zur Unterscheidung zwischen einer spontanen und einer stimulierten Herzaktion anhand deren unterschiedlicher Signalformen ausgebildet sind,
**dadurch gekennzeichnet, dass**
die Mittel zur Aufnahme und Auswertung ferner derart ausgebildet sind, dass nach der Aussendung eines Herzreizimpulses im VVI-Modus bei Nichterfassung eines evozierten Potentials eine erste Folge von Herzreizimpulsen mit sprunghaft vergrößertem Impulsabstand ausgesandt, anschließend der Impulsabstand wieder sprunghaft auf den ursprünglichen Wert verringert und eine zweite Folge von Herzreizimpulsen ausgesandt wird, wobei der vorbestimmte Zeitraum der Aufnahme und Auswertung innerhalb der zweiten Folge festgelegt wird.

2. Schrittmacheranordnung nach Anspruch 1, **dadurch gekennzeichnet, dass** die Frequenzanalyse mit anschließender Bewertung einen Amplitudenvergleich der Signalanteile des Herzaktionspotentials bei mindestens zwei vorbestimmten Frequenzen oder in mindestens zwei Frequenzbereichen einschließt.

3. Schrittmacheranordnung nach Anspruch 1, **dadurch gekennzeichnet:, dass** die Amplitudendiskriminierung mit anschließender Bewertung eine Unterscheidung zwischen spontaner Herzaktion oder evoziertem Potential anhand der absoluten oder relativen Häufigkeit des Auftretens von Amplitudenwerten unterhalb der Referenzspannung einschließt.

4. Schrittmacheranordnung nach Anspruch 3, **dadurch gekennzeichnet, dass** die Aufnahme- und Auswertungsschaltung (1a) aufweist:
- eine Austastschaltung (11) zum Ausblenden von Signalen unmittelbar nach dem Herzreizimpuls,
- einen Vorverstärker (12),
- einen programmierbaren Messverstärker (13),
- ein Bandfilter (14) mit einem Durchlassbereich von 0,5 bis 10 Hz,
- eine Vergleichereinheit (15) zur Amplitudendiskriminierung des Messsignals,
- einen A/D-Wandler und
- eine logische Verarbeitungseinheit zur Analyse der Vergleichsergebnisse entsprechend dem Verfahrensablauf.

5. Schrittmacheranordnung nach einem der vorangehenden Ansprüche, **dadurch gekennzeichnet, dass** das Messzeitfenster eine Breite von 60 ms hat und sein Beginn zwischen 150 und 250 ms nach dem Herzreizimpuls in Schritten einstellbar ist.

6. Schrittmacheranordnung nach einem der vorangehenden Ansprüche, **dadurch gekennzeichnet, dass** die Abtastrate für die einzelnen Messungen innerhalb des Messzeitfensters größer als 100 Hz ist.

7. Schrittmacheranordnung nach einem der vorangehenden Ansprüche, **dadurch gekennzeichnet, dass** die Steuereinheit (1b; IC; MC) bei Feststellung fehlender Effektivität eines anfänglich ausgesandten Herzreizimpulses die Aussendung eines folgenden Herzreizimpulses mit auf einen ersten Sicherheits-Energiewert erhöhter Impulsenergie und die Feststellung von dessen Effektivität steuert, wobei bei erneuter Feststellung fehlender Effektivität ein nächster Herzreizimpuls mit auf einen zweiten Sicherheits-Energiewert erhöhter Impulsenergie ausgesandt wird.

8. Schrittmacheranordnung nach Anspruch 7, **dadurch gekennzeichnet, dass** dem ersten Sicherheits-Energiewert eine Spannung von 4,8 V und dem zweiten Sicherheits-Energiewert eine Spannung von 9,6 V entspricht.

9. Schrittmacheranordnung nach einem der vorangehenden Ansprüche, **dadurch gekennzeichnet, dass** die Mittel zur Aufnahme- und Auswertung (1a; TF; TA; IC) und die Steuereinheit (1b; IC; MC) implantierbar sind und letztere von außerhalb des Körpers, insbesondere über einen Reed-Schalter (RS), aktivierbar ist.

## Claims

1. A pacemaker arrangement comprising means (P) for emitting a heart pacing pulse with a predetermined pulse energy to a heart, means (1a; TF; TA; IC) for recording and evaluating a heart action potential in dependence on time in the heart within a predetermined period after emission of the heart pacing pulse, wherein the detection of a stimulated heart action indicates the effectiveness of the emitted heart pacing pulse and the absence thereof indicates lack of effectiveness of the heart pacing pulse, and a control unit (1b; IC; MC), wherein the means for recording and evaluating the heart action potential are adapted for the intraventricular recording of a plurality of voltage values at time spacings within a measurement time window established in the predetermined period of time and for a comparison with a predetermined signal configuration with the inclusion of amplitude discrimination in respect of the signal in the time or frequency domain in relation to a predetermined reference voltage with subsequent evaluation, for distinguishing between a spontaneous and a stimulated heart action on the basis of the differing signal shapes thereof,
**characterised in that**
the recording and evaluation means are further so adapted that after the emission of a heart pacing pulse in the VVI mode upon non-detection of an evoked potential a first series of heart pacing pulses with an abruptly increased pulse spacing is emitted, then the pulse spacing is again abruptly reduced to the original value and a second series of heart pacing pulses is emitted, wherein the predetermined period of time of recording and evaluation is established within the second series.

2. A pacemaker arrangement according to claim 1 **characterised in that** frequency analysis with subsequent assessment includes amplitude comparison of the signal components of the heart action potential at at least two predetermined frequencies or in at least two frequency ranges.

3. A pacemaker arrangement according to claim 1 **characterised in that** amplitude discrimination with subsequent assessment includes a distinction between spontaneous heart action or evoked potential on the basis of the absolute or relative frequency of the occurrence of amplitude values below the reference voltage.

4. A pacemaker arrangement according to claim 3 **characterised in that** the recording and evaluation circuit (1a) has:
- a blanking circuit (11) for cutting out signals immediately after the heart pacing pulse,
- a pre-amplifier (12),
- a programmable measurement amplifier (13),
- a band filter (14) with a band-pass range of 0.5 to 10 Hz,
- a comparator unit (15) for amplitude discrimination in respect of the measurement signal,
- an A/D-converter, and
- a logic processing unit for analysis of the comparison results in accordance with the operating procedure.

5. A pacemaker arrangement according to one of the preceding claims **characterised in that** the measurement time window is of a width of 60 ms and its beginning can be adjusted in steps at between 150 and 250 ms after the heart pacing pulse.

6. A pacemaker arrangement according to one of the preceding claims **characterised in that** the sample rate for the individual measurements within the measurement time window is greater than 100 Hz.

7. A pacemaker arrangement according to one of the preceding claims **characterised in that** upon the establishment of lack of effectiveness of an initially emitted heart pacing pulse the control unit (1b; IC; MC) controls the emission of a following heart pacing pulse with a pulse energy which is increased to a first safety energy value and the establishment of the effectiveness thereof, wherein when a lack of effectiveness is established again a next heart pacing pulse with a pulse energy which is increased to a second safety energy value is emitted.

8. A pacemaker arrangement according to claim 7 **characterised in that** a voltage of 4.8 V corresponds to the first safety energy value and a voltage of 9.6 V corresponds to a second safety energy value.

9. A pacemaker arrangement according to one of the preceding claims **characterised in that** the recording and evaluation means (1a; TF; TA; IC) and the control unit (1b; IC; MC) are implantable and the latter is activatable from outside the body, in particular by way of a reed switch (RS).

## Revendications

1. Stimulateur cardiaque, comprenant des moyens (P) pour émettre une impulsion de stimulation cardiaque avec une énergie d'impulsion prédéterminée à destination d'un coeur, des moyens (1a ; TF ; TA ; IC) pour enregistrer et pour évaluer un potentiel d'action cardiaque en fonction du temps dans le coeur pendant un intervalle de temps prédéterminé après émission de l'impulsion de stimulation cardiaque, la détection d'une action cardiaque stimulée indiquant l'efficacité de l'impulsion de stimulation cardiaque émise cependant que son absence indique un manque d'efficacité de l'impulsion de stimulation cardiaque, et une unité de commande (1b ; IC ; MC), lesdits moyens pour enregistrer et pour évaluer le potentiel d'action cardiaque étant agencés pour enregistrer par voie intraventriculaire plusieurs valeurs de tension à des intervalles de temps pendant une fenêtre temporelle de mesure fixée dans une période prédéterminée et pour exécuter une comparaison avec une évolution de signal prédéterminée tout en incluant une discrimination d'amplitude du signal dans le domaine temporel ou dans le domaine des fréquences par référence à une tension de référence prédéterminée, avec une exploitation consécutive en vue de faire une distinction entre une action cardiaque spontanée et une action cardiaque stimulée à l'aide de leurs formes de signaux différentes,
**caractérisé en ce que** les moyens d'enregistrement et d'évaluation ne sont en outre ainsi réalisés qu'après l'émission d'une impulsion de stimulation cardiaque dans le mode VVI lors de l'absence de détection d'un potentiel appelé, une première suite d'impulsions de stimulation cardiaque, dont l'écart réciproque est accru par saut, est émise, puis l'écart entre impulsions est de nouveau réduit par saut à la valeur initiale et une seconde suite d'impulsions de stimulation cardiaque est émise, la période prédéterminée pour l'enregistrement et l'évaluation étant fixée à l'intérieur de la seconde suite.

2. Stimulateur cardiaque selon la revendication 1, **caractérisé en ce que** l'analyse de fréquence avec évaluation successive inclut une comparaison de l'amplitude des composantes du signal du potentiel d'action cardiaque pour au moins deux fréquences prédéterminées ou dans au moins deux plages de fréquences.

3. Stimulateur cardiaque selon la revendication 1, **caractérisé en ce que** la discrimination d'amplitude avec évaluation successive inclut une différenciation entre une action cardiaque spontanée ou un potentiel appelé sur la base de la fréquence absolue ou relative de l'apparition de valeurs d'amplitude au-dessous de la tension de référence.

4. Stimulateur cardiaque selon la revendication 3, **caractérisé en ce que** le circuit d'enregistrement et d'évaluation (1a) comprend :
-- un circuit de suppression (11) pour occulter des signaux immédiatement après l'impulsion de stimulation cardiaque,
-- un préamplificateur (12),
-- un amplificateur de mesure programmable (13),
-- un filtre passe-bande (14) avec une plage de transmission de 0,5 à 10 Hz,
-- une unité de comparaison (15) pour la discrimination d'amplitude du signal de mesure,
-- un convertisseur A/N, et
-- une unité de traitement logique pour l'analyse des résultats de comparaison en fonction du déroulement du procédé.

5. Stimulateur cardiaque selon l'une des revendications précédentes, **caractérisé en ce que** la fenêtre temporelle de mesure a une largeur de 60 millisecondes, et **en ce que** son commencement est réglable entre 150 et 250 millisecondes après l'impulsion de stimulation cardiaque, par incréments.

6. Stimulateur cardiaque selon l'une des revendications précédentes, **caractérisé en ce que** la cadence d'échantillonnage pour les mesures individuelles pendant la fenêtre temporelle de mesure est supérieure à 100 Hz.

7. Stimulateur cardiaque selon l'une des revendications précédentes, **caractérisé en ce que**, lors d'une détermination d'un manque d'efficacité d'une impulsion de stimulation cardiaque initialement émise, l'unité de commande (1b ; IC ; MC) commande l'émission d'une impulsion de stimulation cardiaque suivante avec une énergie d'impulsion accrue à une première valeur d'énergie de sécurité et la détermination de cette efficacité, et lors d'une nouvelle détermination d'un manque d'efficacité, une impulsion de stimulation cardiaque suivante est émise avec une énergie d'impulsion accrue à une seconde valeur énergétique de sécurité.

8. Stimulateur cardiaque selon la revendication 7, **caractérisé en ce qu'**une tension de 4,8 volts correspond à la première valeur d'énergie de sécurité, et une tension de 9,6 volts correspond à la seconde valeur d'énergie de sécurité.

9. Stimulateur cardiaque selon l'une des revendications précédentes, **caractérisé en ce que** les moyens d'enregistrement et d'évaluation (1a ; TF ; TA ; IC) et l'unité de commande (1b ; IC ; MC) sont implantables, et **en ce que** ladite unité de commande peut être activée depuis l'extérieur du corps, en particulier par l'intermédiaire d'un commutateur Reed (RS).
